(19) Europäisches Patentamt
European Patent Office
Office européen des brevets

(11) EP 1 627 920 A1

# (12) EUROPEAN PATENT APPLICATION

(43) Date of publication:
**22.02.2006 Bulletin 2006/08**

(51) Int Cl.:
***C12N 15/57*** *(2006.01)* ***C12N 9/76*** *(2006.01)*
***C12M 1/00*** *(2006.01)*

(21) Application number: **05021863.5**

(22) Date of filing: **24.01.2001**

(84) Designated Contracting States:
**AT BE CH CY DE DK ES FI FR GB GR IE IT LI LU MC NL PT SE TR**

(30) Priority: **24.01.2000 US 177348 P**

(62) Document number(s) of the earlier application(s) in accordance with Art. 76 EPC:
**01911524.5 / 1 250 442**

(71) Applicant: **Polymun Scientific Immunbiologische Forschung GmbH**
**1190 Wien (AT)**

(72) Inventors:
- **Mattanovich, Diethard**
**1120 Vienna (AT)**
- **Katinger, Hermann**
**1190 Vienna (AT)**
- **Hohenblum, Hubertus**
**1140 Vienna (AT)**
- **Naschberger, Stefan**
**1170 Vienna (AT)**
- **Weik, Robert**
**1030 Vienna (AT)**

(74) Representative: **Bogensberger, Burkhard**
**BOGENSBERGER**
**Patent- & Markenbüro**
**Austrasse 79**
**9490 Vaduz (LI)**

Remarks:
This application was filed on 07.10.2005 as a divisional application to the application mentioned under INID code 62.

## (54) Method for the manufacture of recombinant trypsin

(57) The present invention relates to an improved method and apparatus for carrying out protein refolding specifically useful for processing trypsinogen that has accumulated intracellularly in the form of inclusion bodies.

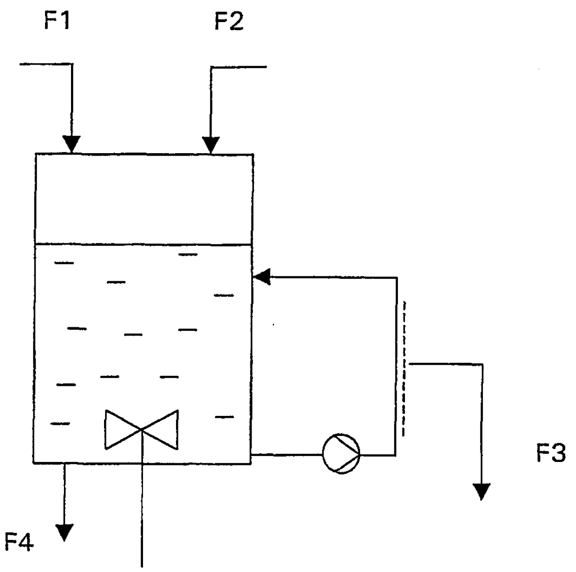


Fig. 1

EP 1 627 920 A1

**Description**

TECHNICAL FIELD

The present invention is in the field of molecular biology and biotechnology and relates to methods for the manufacture of recombinant trypsinogen and trypsin, respectively.

BACKGROUND OF THE INVENTION

The protease trypsin is used in biotechnology for different purposes, e.g. to detach adherent cells from surfaces, to enhance viral infection during virus production, or to process fusion proteins. Today, trypsin prepared from animal, particularly bovine and porcine tissue is still in widespread use. As it is a general desire and tendency to replace material, e.g., proteins, extracted from animal sources by equivalents made by recombinant technology it is an objective of the present invention to provide for a method to efficiently produce recombinant trypsin of high purity and in high amounts.

As is known in the art, the proteolytic activity of trypsin poses two major obstacles for recombinant production thereof:

> Trypsin activity seriously damages the host cell. Therefore the direct production of active trypsin yields very low expression levels (Yee L. and Blanch H.W., 1992, Biotechnol. Bioeng. 41, 781-790). To circumvent this problem, the inactive precursor trypsinogen may be expressed instead of the mature trypsin. Trypsinogen is, however, susceptible to digestion by trypsin and other proteases (Abita J.P. et al. 1969, European J. Biochem. 8, 314-324) resulting in its cleavage to active trypsin. It is further known in the art that trypsin may act on itself and autocatalyze its own cleavage to finally become inactivated.

In this context, WO97/00316 reports that the recombinant production of trypsinogen by a filamentous fungus even when secreted into the medium by the host cell will yield at least some mature trypsin due to either automaturation or maturation by proteases produced by the host cell.

EP 597681 reports methods for the construction of suitable vectors for recombinant production of trypsin or trypsinogen in an E.coli expression system. They also teach that conversion of trypsinogen to trypsin is accomplished by enteropeptidase.

EP 597681 is silent with regard to culturing the successfully transformed E.coli cells on another than a solid agar medium and thus do not indicate any advantages or disadvantages of this procaryotic expression system. On the other hand, the methods disclosed in WO97/00316 allegedly yield a several fold increased trypsin level as compared to those apparent from other microbial systems, without providing any basis for such a comparison, however. WO97/00316 also teaches that the expression of trypsins, especially mammalian trypsins, is accomplished to only extremely low levels in the art.

The presence of two protein species (e.g., trypsinogen and trypsin) in a bacterial or eukaryotic cell culture broth will likely lead to complications during product recovery and purification, because both proteins need to be harvested and purified. Moreover, in case of microbial trypsinogen secretion the simultaneous presence of trypsin will accelerate the maturation of trypsinogen to trypsin and, subsequently, may lead to at least a partial inactivation of the desired final product trypsin. Even worse, it may additionally damage the host cells resulting in a reduction of the biomass and product yield and to a higher load of undesired contamination by host cell constituents.

WO 00/17332 reports a method for the production of trypsinogen using a modified trypsinogen anlog that cannot be autocatalytically cleaved during incubation. The method requires the addition of a diaminopeptidase for activation of trypsinogen to trypsin after separation of the trypsin analog from the cell culture medium.

On the other hand, WO 99/10503 reports a method for the production of trypsinogen, wherein a synthetic autocatalytic cleavage site is introduced to the trypsinogen molecule that does not occur naturally and which replaces the naturally occurring autocleavage site. It is explicitly desired therein that the zymogen, i.e., the trypsinogen, is autocatalytically active, at least to a low degree. The host cells producing the zymogen are not harmed by the proteolytic activitiy of the zymogen, because the zymogen is accumulated intracellulary in the form of inactive protein aggregates, i.e. inclusion bodies.

All of the aforementioned methods have in common that they require either a step of modifying the trypsinogen molecule in order to increase its autocatalytic activity or, on the contrary, to prevent undesired impact on the producing cells, or require a step of adding a proteinase for activating the zymogen, e.g., trypsinogen trypsinogen to the active enzyme, e.g., trypsin, which then of course further requires removing the proteinase from the final product unless the added proteinase is trypsin.

Therefore it is an objective of the present invention to provide a simple yet highly efficient method suitable for the production of trypsin by recombinant technology without the drawbacks known from the prior art processes.

## BRIEF DESCRIPTION OF THE INVENTION

The objective is reached according to the present invention by a method of manufacturing recombinant trypsin or trypsinogen, respectively, during which no unintended trypsin activity nor activation of trypsinogen to trypsin occurs.

In one embodiment, a trypsinogen gene (e.g. human trypsinogen 1) is inserted into yeast expression vectors in a way such that a sequence encoding a secretion leader peptide is included (e.g. pPICZαB for the yeast *Pichia pastoris*), and the recombinant vectors are introduced into appropriate host cells. The cells are cultivated for expression and the recombinant trypsinogen can then be found in the supernatant of the culture, from where it is isolated, purified and activated to trypsin. This procedure was found to be superior to all other expression systems for trypsinogen tested or known to us so far, as it revealed the following important advantages:

- the expression level was high;
- the trypsinogen protein secreted into the nutrient medium was correctly folded and could be directly activated to trypsin;
- no unintended activation to trypsin occurred during the production process.

In another embodiment, a trypsinogen gene is inserted into an expression vector without a secretion leader sequence; so that the product is accumulated inside the host cell, such as a bacterial cell. The trypsinogen forms insoluble aggregates that can be released by breaking up the cells, and harvested by centrifugation, filtration or other methods. To solubilize the recombinant protein, a suitable procedure for denaturing and refolding is applied, preferably by a continuous method as described hereinbelow.

Further embodiments of the present invention are described in the dependent claims.

## BRIEF DESCRIPTION OF THE FIGURES

Fig. 1 shows a schematic setup of the refolding device or arrangement as described herein.

## DETAILED DESCRIPTION OF THE INVENTION:

The present invention relates to production systems for recombinant trypsin or trypsinogen.
In one embodiment a gene for trypsinogen is introduced into yeast so that the gene product is secreted into the culture supernatant. The yeast can be a methylotrophic yeast (e.g. *Pichia pastoris, P. methanolica* or *Hansenula polymorpha*), or *Saccharomyces cerevisiae, Kluyveromyces lactis* or another yeast. The expression system comprises a promoter (e.g. alcohol oxidase, alcohol dehydrogenase, glyceraldehyde-3-phosphate dehydrogenase or others), a secretion leader (e.g. yeast α-factor, yeast phosphatase, from mammalian or plant source, from trypsinogen, or others), a transcription terminator (e.g. alcohol oxidase, alcohol dehydrogenase or others), and a selective marker (e.g. an amino acid synthesis gene or an antibiotic resistance gene). The recombinant gene and one or more of the additional elements described above are introduced into the yeast cell by e.g. electroporation, treatment with lithium acetate or lithium chloride, preparation of spheroplasts and treatment with calcium salts, or other suitable methods. The yeasts are then cultivated under conditions appropriate for the expression of trypsinogen, and the product is harvested with the culture supernatant.

The product can be further purified from the culture supernatant with a strong cation exchange resin. In most procedures known in the art, the culture supernatant is being exchanged and turned into a buffer having a low conductivity by, e.g., cross flow filtration, dialysis, etc. prior to being loaded onto a gel chromatography column. However, when the culture supernatant is converted to a sodium acetate/acetic acid buffer, only small amounts of trypsinogen contained therein adhere and bind to the cation exchange resin. Therefore a new method is proposed herein, which comprises adding $Ca^{2+}$ ions to the solution that contains the desired product (e.g., trypsinogen, modified trypsinogen, or trypsinogen-homologue) before it is supplied to the gel chromatography column. This can be accomplished by the addition of a soluble calcium salt such as, for example, $CaCl_2$ to said solution at an effective concentration of about 0.5 - 400, preferably 1 - 300, most preferably about 5 - 20 mmol per 1 liter (1 mM). Using this procedure, the trypsinogen-binding capacity of the resin can be augmented by a factor of at least 3, typically by a factor of 3 to 6. The lower limit of $Ca^{2+}$ concentration in the feed solution is primarily determined by the ability of trypsinogen to get activated to trypsin, which ability fades away if the feed solution falls short of $Ca^{2+}$. Under the preferred procedural conditions of the present invention (see Example 5) the minimum $Ca^{2+}$ concentration in the feed solution and preferably also in the washing and eluting buffers has been found to be approximately 1 mM.

The upper limit is basically determined by the degree of conductivity in the feed solution. It was found that in case the conductivity exceeds a certain level (due to the addition of calcium salt), the benefit of increased affinity of

($Ca^{2+}$)-loaded trypsinogen for binding to the chromatography resin gradually fades away. Under the preferred procedural conditions of the present invention (see Example 5) the maximum $Ca^{2+}$ concentration should not exceed 300 mM. For most applications, a $CaCl_2$ concentration of 5 to 20 mM will be best.

Thus the present purification method improves the overall economy and cost-effectiveness of known state-of-the-art purification processes due to an at least threefold increase in binding capacity of the column (which means that at least thrice the usual amount of trypsinogen is bound per volume of resin) and, additionally, due to a significant augmentation of recovery of activatable trypsinogen.

The best results according to the present invention were obtained in a procedure, wherein the harvested culture supernatant, after removal of solids and after addition of an effective amount of $CaCl_2$, was directly, i.e. without a buffer exchanging step, loaded onto the column, and wherein each washing and eluting buffer contained an effective amount of $CaCl_2$. While $Ca^{2+}$ in the feed solution improves binding to the cation exchange resin, $Ca^{2+}$ in the washing and eluting buffers facilitates subsequent activation of trypsinogen to trypsin.

The methods for production of trypsin or trypsinogen that have been described in the art so far either yield very low amounts of the product or cause at least partial cleavage of trypsinogen during the cultivation. This is not the case, however, with the yeast method described herein, which makes this method superior to the hitherto known processes of trypsin production.

In another embodiment of the present invention a gene for trypsinogen is introduced into cells in a way such that the gene product remains in the cytoplasm. The cells can be bacteria (e.g. *Escherichia coli*), yeasts (e.g. *P. pastoris, S. cerevisiae* or others), or any other recombinant host cell. The expression system comprises a promoter (e.g. lac, tac, trc, phage T7 $\phi$10, phage $\lambda$ $P_L$ or $P_R$, alcohol oxidase, alcohol dehydrogenase, glyceraldehyde-3-phosphate dehydrogenase, or others), a transcription terminator (e.g. rrnB, alcohol oxidase, alcohol dehydrogenase, or others), and a selective marker (e.g. an amino acid synthesis gene or an antibiotic resistance gene).

The recombinant gene together with at least some of the additional elements described above are introduced into the cell by e.g. electroporation, treatment with lithium acetate or lithium chloride, preparation of spheroplasts and treatment with calcium salts, or other suitable methods. The cells are then cultivated under conditions appropriate for the expression of trypsinogen. Then the cells are harvested e.g. by centrifugation and disintegrated mechanically (e.g. pressure, ultrasound) or chemically (e.g. cell wall degrading enzymes, detergents or others). The aggregated trypsinogen accumulated inside the cells in the form of inactive inclusion bodies can be partially purified by washing and subsequently subjected to solubilization by treatment with a chaotropic agent (e.g. urea or guanidinium hydrochloride). The protein gets denatured by this treatment. To attain a correctly folded protein, the denaturing agent must then be removed. This can be accomplished, e.g., by dialysis, dilution or diafiltration. For some proteins, as is the case with trypsinogen, the process of refolding is greatly enhanced if the free cystein residues of the denatured protein are blocked by the formation of mixed disulfides with glutathione or by S-sulfonation. This is accomplished by the treatment of the denatured protein solution with oxidized glutathione or with $SO_3^{2-}$ and 2-nitro-5-(thiosulpho)-benzoate in the presence of a denaturing concentration of the chaotropic agent. After removal of the blocking solution, refolding is performed by removing the chaotropic agent, as described above.

The refolding procedure for trypsinogen is preferably performed by a new method which is also a part of the present invention. This method is not limited to the refolding of trypsinogen but is also applicable for refolding any denatured protein. It is a semi-continuous or continuous method and requires a reactor unit with the following minimal features (Fig.1): a device for mixing, a connection to a diafiltration (or another equivalent filtration) circuit, an inlet for feeding the sample (i.e., the denatured protein), an inlet for feeding renaturation buffer, and an outlet for draining off product (i.e., renaturing and/or renatured protein).

The operation of this apparatus comprises the steps of feeding denatured protein to the reactor, removing denaturing agents via the diafiltration circuit and removing processed protein solution from the reactor. The reactor can be operated in a continuous or semi-continuous way. In the semi-continuos mode, feed into and volume removal from the reactor unit is done intermittently, e.g. in predetermined intervals depending upon the refolding properties of the treated protein. In continuous mode operation, the influxes and effluxes are adjusted to the refolding velocity of the concerned protein in order to maintain a desired dynamic steady state equilibrium within the reactor. While it is preferred that the flow rates are adjusted such as to allow for sufficient residence time of the denatured protein for substantially complete refolding (e.g. 95%) inside the reactor unit, it may be desired in some applications to adjust the flow rates such as to increase the throughput of the material while reducing the mean residence time of the protein inside the unit and to drain off protein whereof a substantial, optionally prevailing, portion is still in the process of refolding, thus allowing completion of refolding of that portion of denatured protein outside the reactor unit.

Apart from refolding denatured proteins, the apparatus can also be used for other treatments of protein solutions, e.g., for a continuous buffer exchange, or for blocking cystein residues, as described above.

The operation conditions for refolding are characterized as follows:

F1 = Flux 1, feed denatured protein

F2 = Flux 2, feed refolding buffer
F3 = Flux 3, outlet diafiltrate
F4 = Flux 4, outlet refolded protein

By definition, F1 and F2 have positive values or 0 (zero), and F3 and F4 negative values or 0 (zero). Absolute values of F3 or F4 are indicated as |F3| or |F4|.

If constant volume and constant protein concentration in the reactor are desired, following conditions must be applied:

$$F1 + F4 = 0 \quad \text{and} \qquad (1)$$

and

$$F2 + F3 = 0 \qquad (2)$$

For constant volume and increasing protein concentration:

$$F1 + F2 = |F3 + F4|, \quad \text{with } F1 > |F4| \text{ and } |F3| > F2 \qquad (3)$$

For increasing volume and increasing protein concentration:

$$F1 > |F4| \text{ and } (F1 + F2) > |F3 + F4|, \text{ e.g. } F2 = |F3| \qquad (4)$$

Generally, the concentration of denaturing agent will not be reduced to zero, but to an equilibrium level depending mainly on the flux levels. A certain low concentration of chaotropic agent is in most cases beneficial to the refolding rate. Based on the assumption of a constant volume and no retention of the denaturing agent at the diafiltration membrane (as is the case with, e.g., urea as denaturing agent), the following condition holds for an equilibrium state:

$$C_{F1} \times F1 + C_{F2} \times F2 + C_{F3} \times F3 + C_{F4} \times F4 = 0 \qquad (5)$$

$C_{Fi}$ .......... concentration of denaturing agent at flux i (i = 1 to 4)

In an equilibrium state, $C_{F3} = C_{F4} = C_R$ (concentration in the reactor). If one assumes that F1 = -F4 and $C_{F2}$ = 0, it follows that:

$$F3 / F1 = -(C_{F1} - C_R) / C_R \qquad (6)$$

Following equation (6), the relation of F3 to F1 can be calculated based on the given value of $C_{F1}$ and the desired value of $C_R$.

Generally, F1 should be adjusted to the refolding rate of the protein. Therefore, depending on the respective refolding rate of a protein, values of F1 between 0.02 and 4 $h^{-1}$ are appropriate and preferred. Lower flow rates, though technically possible without negative effect to the refolding as such, are not preferred as they slow down the overall refolding process and thus reduce the process economy.

The main advantages of the refolding method according to the present invention comprise the following:

- the reactor size can be much smaller than with batch processes;
- for refolding in a batch system, the concentration of denatured protein (and of denaturing agent fed together with the denatured protein) must be kept sufficiently low (e.g., at a level of maximum 10-50 μg protein per ml renaturation buffer) to avoid protein aggregation, which requires providing a high starting volume of renaturing buffer in the batch system;
- the protein concentration can be substantially increased when the feeding rate of denatured protein is equal or lower than the refolding rate, while the removal of denaturing agent at least equals its introduction via the denatured protein feed;

- higher protein concentrations means smaller volumes for storage and reaction vessels and less consumption of chemicals (e.g. denaturing agents, buffer, cystein residue blocking agents);
- higher product yield is achievable than with batch processes;
- aseptic operation can easily be accomplished;
- considerable reduction of processing hence product costs.

In order that the invention described herein may be more fully understood, the following examples are set forth. The examples are for illustrative purposes and are not to be construed as limiting this invention in any respect. Particularly, it is understood that the procedures of the present invention, though mainly exemplified in the subsequent Examples for recombinant human trypsinogen 1 and its corresponding trypsin, are also applicable without inventive input to other human trypsinogens and the corresponding trypsins as well as to trypsinogens and corresponding trypsins of non-human origin, including bovine and porcine trypsinogens and trypsins. It is further understood that the present invention shall also comprise variations of the expressly disclosed embodiments to an extent as would be contemplated by a person of ordinary skill in the art.

Example 1: Expression of human trypsinogen 1 in the yeast *Pichia pastoris*

The gene for human trypsinogen 1 (Gene 41, 305-310, 1986; Swissprot locus TRY1_HUMAN, accession no. P07477; for the sequence see Table 1) was inserted into the vector pPICZαB (Invitrogen), which was previously digested with SfiI.

Table 1: Nucleotide sequence of the human trypsinogen 1 gene beginning at the propeptide sequence (SEQ ID NO 1)

| |
|---|
| GCCCCCTTTGATGATGATGACAAGATCGTTGGGGGCTACAACTGTGAGGAG<br>AATTCTGTCCCCTACCAGGTGTCCCTGAATTCTGGCTACCACTTCTGTGGTG<br>GCTCCCTCATCAACGAACAGTGGGTGGTATCAGCAGGCCACTGCTACAAGT<br>CCCGCATCCAGGTGAGACTGGGAGAGCACAACATCGAAGTCCTGGAGGGG<br>AATGAGCAGTTCATCAATGCAGCCAAGATCATCCGCCACCCCCAATACGAC<br>AGGAAGACTCTGAACAATGACATCATGTTAATCAAGCTCTCCTCACGTGCA<br>GTAATCAACGCCCGCGTGTCCACCATCTCTCTGCCCACCGCCCCTCCAGCC<br>ACTGGCACGAAGTGCCTCATCTCTGGCTGGGGCAACACTGCGAGCTCTGGC<br>GCCGACTACCCAGACGAGCTGCAGTGCCTGGATGCTCCTGTGCTGAGCCAG<br>GCTAAGTGTGAAGCCTCCTACCCTGGAAAGATTACCAGCAACATGTTCTGT<br>GTGGGCTTCCTTGAGGGAGGCAAGGATTCATGTCAGGGTGATTCTGGTGGC<br>CCTGTGGTCTGCAATGGACAGCTCCAAGGAGTTGTCTCCTGGGGTGATGGC<br>TGTGCCCAGAAGAACAAGCCTGGAGTCTACACCAAGGTCTACAACTACGTG<br>AAATGGATTAAGAACACCATAGCTGCCAATAGCTAA |

Table 2: PCR primers for cloning into pPICZαB

| |
|---|
| Forward primer (SEQ ID NO 2):<br>5' - CATGCCATGACTCGCGGCCCAGCCGGCCCCCTTTGATGATGATG - 3' |
| Backward primer (SEQ ID NO 3):<br>5' - CATGCCATGACTCGCGGCCGGCTGGGCCTTAGCTATTGGCAGCTATGG - 3' |

The corresponding SfiI sites were attached to the trypsinogen gene by PCR (primer sequences see Table 2). The recombinant plasmid was transformed into *P. pastoris* strain GS115 or strain X33, respectively, by electroporation. Positive yeast clones were selected by resistance to zeocin. Several clones were cultivated in 250 ml baffled shake flasks. As cultivation media, either buffered minimal medium (100 mM potassium phosphate, pH 6.0; 1.34 % YNB; $4\times10^{-5}$ % biotin; 1 % glycerol or 0.5 % methanol) or buffered complex medium (1 % yeast extract; 2 % peptone; 100 mM potassium phosphate, pH 6.0; 1.34 % YNB; $4\times10^{-5}$ % biotin; 1 % glycerol or 0.5 % methanol) was used. For induction of recombinant gene expression, the cells were centrifuged and resuspended in fresh medium containing methanol instead of glycerol. Then the cells were removed from the supernatant by centrifugation, and the recombinant trypsinogen was detected in the supernatant by SDS polyacrylamide gel electrophoresis. This procedure enabled the selection of strong overproducing clones. Activation of trypsinogen was not observed in these cultures.

Alternatively, the gene for human trypsinogen 1 (sequence see Table 1) was inserted into the vector pHILS1 (Invitrogen), which was previously digested with XhoI and BamHI. The corresponding XhoI and BamHI sites were attached to the trypsinogen gene by PCR (primer sequences see Table 3). The recombinant plasmid was transformed into *P. pastoris* strain GS115 by the protoplast transformation method. Positive yeast clones were selected by growth on minimal plates without histidin. Cultivation and analysis followed the procedures described above.

Table 3: PCR primers for cloning into pHILS1

| |
|---|
| Forward primer (SEQ ID NO 4):<br>5' - CCGCTCGAGCCCCCTTTGATGATGATG - 3' |
| Backward primer (SEQ ID NO 5):<br>5' - GCGGATCCTTAGCTATTGGCAGCTATGG - 3' |

Both methods yielded secretion of correctly folded trypsinogen into the culture medium. Refolding was not necessary which rendered this yeast-based process for the manufacture of trypsinogen relatively simple and well suitable for industrial scale production, though optimization of the culturing conditions may be useful to further improve the product yield. The yield of secreted trypsinogen in the present experiments was lower than in the E.coli system (e.g., as a maximum only about 10% of the yield achieved with E.coli), and typically amounted to at least 10 mg/l in the shake flask culture at a biomass concentration of approximately 10 g/l (calculated as dry mass per liter of culture broth) and at least 30 mg/l in an agitated lab-scale fermenter vessel at a biomass concentration (calculated as dry mass) of approximately 30 g/l. As the present process still leaves room for optimization it is expected that a biomass concentration of up to 100 g/l (as reported in literature for various P.pastoris fermentation processes) and, correspondingly, a trypsinogen concentration of about 0.1 g/l in the culture supernatant may well be reached by changing some parameters of the nutrient medium and the culturing conditions alone.

Taking into account that in our experiments the culture supernatant contained only very few other proteins which considerably facilitates separation and purification (only one or two purification steps are required), and further that no denaturing or renaturing steps were necessary (because the secreted protein was already correctly folded) the final overall product yield obtainable by the yeast method was not so far from the one achieved by the E.coli method.

It is also emphasized that under the aforementioned expression and culturing conditions, secreted trypsinogen remained stable in the culture broth during several days of cultivation. No autocatalytic activation to trypsin was observed (probably due to the acidic pH of the cell culture suspension being in a range of from pH 2 to pH 7.5, which is far from being optimal for autocatalytic conversion of trypsinogen) nor any negative impact on cell viability. Thus, the avoidance of various undesired procedural developments including but not limited to uncontrolled trypsin formation, inhibition of cell growth, acceleration of cell lysis, trypsin autodigestion, supernatant pollution by cell components of lysed cells, additional purification steps, product contamination, product loss, etc. is believed to confer a considerable advantage of the present invention over the state-of-the-art technology.

Example 2: Expression of human trypsinogen 1 in *Escherichia coli*

The expression vector pET3a (Studier et al. 1 990) was digested either with NdeI and BamHI or with BamHI alone, and the human trypsinogen 1 gene was inserted. The corresponding restriction sites were attached by PCR (primer sequences see Table 4). The recombinant plasmid was transferred into *E. coli* K12 HMS174(DE3) by electroporation. Positive clones were selected by growth on ampicillin plates.

Unexpectedly, insertion of the gene for human trypsinogen 1 into the expression vector right after the start codon did not result in a measurable expression of the desired protein. Only after insertion of an oligonucleotide between the start codon and the trypsinogen gene high expression of trypsinogen was successfully achieved. Best results were obtained when using an oligonucleotide coding for several, preferably at least 10, amino acids, of a protein that is known to be highly expressed in a bacterial host, and particularly, when using a nucleotide sequence coding for the first 10 to 15 amino acids of the amino acid sequence of the phage T7 envelope protein.

Table 4: PCR primers for cloning into pET3a

| | |
|---|---|
| Forward primer (for cloning in the NdeI site):<br>5' - GGAATTCCATATGGCCCCCTTTGATGATGATG - 3' | SEQ ID NO 6 |
| Forward primer (for cloning in the BamHI site):<br>5' - GCGGATCCGCCCCCTTTGATGATGATG - 3' | SEQ ID NO 7 |
| Backward primer:<br>5' - GCGGATCCTTAGCTATTGGCAGCTATGG - 3' | SEQ ID NO 8 |

Several clones were cultivated in baffled shake flasks in M9ZB medium (10 g/l bactotryptone; 5 g/l yeast extract; 5 g/l NaCl; 1 g/l $NH_4Cl$; 3 g/l $KH_2PO_4$; 6 g/l $Na_2HPO_4$; 4 g/l glucose; 0.25 g/l $MgSO_4 \cdot 7H_2O$). When the cultures reached an $OD_{600}$ = 1, 0.4 mg/ml IPTG was added for induction, and cultivation was continued for 4 hours. After cell lysis with lysozyme and Triton X-100 (0.4 % in 10 mM TrisCl pH 8.2; 15 mM $MgCl_2$), recombinant trypsinogen was identified in the insoluble fraction of the lysates by western blotting.

Trypsinogen product yields (before denaturing and refolding) typically amounted to at least 20% by weight of bacterial biomass (calculated as dry mass) of the cell culture. Biomass yields of 20 g dry cell mass per 1 L of cell culture suspension could be achieved in agitated fermenter vessels, resulting in a product yield (trypsinogen) of 4 g/l. In spite of this remarkably high intracellular (i.e., cytoplasmic) accumulation of trypsinogen no negative impact thereof on the producing host cells was observed, which presumably is due to the biosynthesis and intracellular storage of the trypsinogen in the form of inactive aggregated proteins (inclusion bodies).

Example 3: Refolding of human trypsinogen 1

After cell lysis (as described in Example 2), the insoluble fraction was centrifuged and washed two times in 3 - 4 M urea. Then the pellet was resuspended in denaturing solution (9 M urea; 100 mM TrisCl pH 8.6; 1 mM EDTA, 10 mM DTE) and incubated for 2.5 hours at 37°C. To remove DTE, the buffer of the supernatant was changed by ultrafiltration under nitrogen with washing buffer (8 M urea; 100 mM TrisCl pH 8.6; 1 mM EDTA). Then an equal volume of 200 mM oxidized glutathione in 8 M urea was added and the mixture incubated for 3 hours. Then the buffer was changed again with washing buffer.

For renaturation, a reactor unit as described hereinbefore was filled with refolding buffer (50 mM TrisCl pH 8.6; 50 mM $CaCl_2$ and a redox pair (e.g. cystein/cystin; reduced/oxidized glutathione), and refolding buffer was fed at a rate of 0.85 $h^{-1}$. At the same rate filtrate was withdrawn via the diafiltration circuit. Denatured protein was fed at a rate of 0.067 $h^{-1}$. The yield of refolded protein was at least 10 % higher than that achieved with a comparable traditional batch procedure, and usually exceeded the 10% level considerably.

Activation of the purified trypsinogen to trypsin, regardless of the producing host cell system, may be carried out using any of the proteinases known in the art, such as bovine enteropeptidase or trypsins of various origin. It is, however, highly preferred according to the present invention to disregard all of these methods, which would contravene the concept of manufacture of a human recombinant trypsin, and to solely rely on the faint autocatalytic activity of the purified trypsinogen. Moreover, it was found that purely autocatalytic conversion (i.e., without the addition of any protease) of the purified human recombinant trypsinogen manufactured according to the present invention achieved the best product (i.e., active trypsin) yield, particularly when carried out by incubation at 30 to 37 °C, preferably at a slightly alkaline pH between pH 7 and 8, for a period of several hours (e.g., 2-12 hours, depending on the starting amount of trypsinogen). Accordingly, in the preceding Examples 1 and 2 final conversion of trypsinogen to trypsin was carried out by autocatalysis only, i.e., without the addition of any trypsin or other proteinase. Additional information on parameters for autocatalytic conversion of trypsinogen to trypsin may be derived from, e.g., Kay, J. et al., J Biol Chem 246/ 21, 6661-6665, 1971).

Example 4: Refolding of the single chain antibody sc3D6

The feasibility of the refolding procedure was proven with another recombinant protein, a single-chain antibody (sc3D6). An *E. coli* clone expressing sc3D6 was cultivated as described in Example 2.
After cell lysis (according to Example 2), the insoluble fraction was centrifuged and washed two times in 3 - 4 M urea. Then the pellet was resuspended in denaturing solution (9 M urea; 100 mM TrisCl pH 8.6; 1 mM EDTA, 10 mM DTE) and incubated for 2.5 hours at 37°C. To remove DTE, the buffer of the supernatant was changed by ultrafiltration under nitrogen with washing buffer (8 M urea; 100 mM TrisCl pH 8.6; 1 mM EDTA). Then an equal volume of 200 mM oxidized glutathione in 8 M urea was added and the mixture incubated for 3 hours. Then the buffer was changed again with washing buffer.

For renaturation, a reactor unit (as described hereinbefore) was filled with refolding buffer (50 mM TrisCl pH 8.6; 50 mM $CaCl_2$ and a redox pair (e.g. cystein/cystin, or reduced/oxidized glutathione), and refolding buffer was fed at a rate of 3.0 $h^{-1}$. At the same rate filtrate was withdrawn from the diafiltration circuit. Denatured protein was fed at a rate of 0.1 $h^{-1}$. Again, a high yield of refolded protein was achieved.

Example 5: Purification of trypsinogen from yeast culture supernatants

The culture supernatant was cooled and centrifuged. The cell pellet was discarded and the supernatant was centrifuged a second time. The pH of the supernatant was adjusted to 4.0 with acetic acid and 10 mM $CaCl_2$ was added. The supernatant was directly loaded onto a TosoHaas Toyopearl SP550C resin (strong cation exchanger), at a flow rate of 100 cm/h at either room temperature or 4°C, respectively. The column was washed with 50 mM NaAc/Hac + 200 mM NaCl + 10 mM $CaCl_2$. The protein is eluted with 50 mM NaAc/Hac + 400 mM NaCl + 10 mM $CaCl_2$. The recovery yield of trypsinogen, determined by the activity of trypsin, is about 87 %.

Example 6: Virus treatment by recombinantly produced human trypsin 1

Recombinant human trypsin 1 prepared both from E. coli and from P. pastoris as described above was used for the treatment of influenza virus to induce the infectivity of the virus in cell culture. Different strains of influenza virus were compared in Vero/SF cells. The effect of recombinant human trypsin was compared to porcine trypsin, bovine trypsin and bovine chymotrypsin.

Procedure: after removal of the culture supernatant the cells were infected with 50 μl/well of virus dilution (1: 1000; MOI appr. 0.01). After 20 min. incubation at room temperature, 500 μl/well of medium containing two-fold dilutions of different proteases were added. The trypsins of different origins were standardized to the same volumetric activity, measured with TAME (p-toluene-sulfonyl-L-arginine methyl ester) as the substrate. Typical trypsin concentrations were: 0.03 - 1 TAME units per ml of the final solution. One TAME unit is defined as the amount of trypsin hydrolyzing 1 μmol p-toluene-suffonyl-L-arginine methyl ester per minute.

Recombinant human trypsin and porcine trypsin gave identical results and both were superior to bovine trypsin and chymotrypsin.

We claim :

1. A method for the manufacture of recombinant trypsin comprising transforming a host cell with a suitable expression vector that contains a trypsinogen gene, incubating the transformed cells in a suitable cell culture nutrient medium at conditions allowing for cell multiplication and simultaneous or subsequent expression of the trypsinogen gene, separating intracellularly or extracellularly accumulated trypsinogen from the cell suspension, and optionally further processing the separated trypsinogen, characterized in that the trypsinogen gene inserted into the expression vector is identical with a naturally occurring trypsinogen gene.

2. A method according to claim 1, characterized in that the host cell is a eukaryotic cell, and correctly folded trypsinogen produced by said cell accumulates extracellularly in the cell culture medium without significant activation of trypsinogen to trypsin during the period of inubation.

3. A method according to claim 1 or 2, characterized in that incubation of the cell culture is done at a pH of 2 to 7.5.

4. A method according to any one of claims 1 to 3, characterized in that extracellularly accumulated trypsinogen is separated from the cell culture medium comprising the steps of: removing solids from the cell culture medium, adding a $Ca^{2+}$ source to the remaining liquid, optionally adjusting the pH of said liquid, supplying the liquid onto a gel chromatography column and carrying out purification by gel chromatography, preferably by ion exchange chromatography.

5. A method according to any one of claims 1 to 4, characterized in that trypsinogen separated from the cell suspension is further processed comprising a step of autocatalytically activating the trypsinogen to trypsin without an addition of trypsin or of any other proteinase.

6. The method according to any one of claims 1 to 5, characterized in that the trypsinogen is selected from the group consisting of bovine, porcine and human trypsinogen, and preferably is human trypsinogen 1.

7. The method according to any one of claims 2 to 6, wherein the host cell is a yeast selected from the group consisting of Pichia, Saccharomyces, Hansenula and Kluyveromyces, preferably Pichia pastoris.

8. The method according to claim 1, wherein the host cell is E. coli and the trypsinogen is accumulated intracellularly in the form of inactive protein aggregates (inclusion bodies) to a concentration of at least 20% by weight of dry cell mass.

9. The method according to claim 8, wherein the expression vector contains an oligonucleotide sequence encoding a part of a protein that is known to be highly expressed in a bacterial host, which sequence is located between the start codon and the inserted human trypsinogen gene.

10. The method according to claim 8 or 9, wherein accumulated trypsinogen is subjected to denaturing and renaturing steps which are accomplished in a semi-continuous or continuous mode, preferably using a reactor unit equipped with a feed line for feeding denatured protein to the unit, a feed line for feeding renaturation buffer to the unit, a circulation line connected with a diafiltration device for removing denaturing reagent associated with the denatured protein feed, and an outlet for draining off buffer together with refolded protein.

11. A method according to claim 10, characterized in that that its mode of operation is semi-continuous or continuous.

12. An apparatus for continuous or semi-continuous chemical treatment of proteins, comprising a reactor unit that is equipped with a feed line for feeding a protein-containing solution to the unit, a feed line for feeding a solution for chemical treatment of the protein to the unit, a circulation line connected with a diafiltration device for removing undesired ingredients associated with the protein feed, and an outlet for draining off liquid together with treated protein.

13. A method for continuous or semi-continuous refolding of denatured protein, comprising feeding a solution containing denatured protein to the reactor unit defined in claim 12, feeding renaturation buffer to said unit, removing from said unit by diafiltration denaturing agents comprised in the feed solution containing the denatured protein, and draining off from said unit liquid volumes together with refolded protein.

14. The method of claim 13, wherein the method is continuous and the solution containing the denatured protein is fed to the reactor unit at a flow rate F1, renaturation buffer is fed to said unit at a flow rate F2, denaturing agents comprised in the feed solution containing the denatured protein are removed from said unit at a flow rate F3, and liquid volumes containing refolded protein are drained off from said unit at a flow rate F4.

15. The method according to claim 14, wherein the flow rates are adjusted such as to maintain a constant or increasing protein concentration within the reactor unit.

16. The method according to any one of claims 13 to 15, wherein the protein is bovin, porcine or human trypsinogen, preferably recombinant human trypsinogen 1.

17. A method for the separation and/or purification of a trypsinogen or homologue thereof from a solid-free liquid containing said trypsinogen or homologue thereof, characterized in that the liquid is supplemented with a $Ca^{2+}$ source and thereafter supplied to a gel chromtography resin, preferably a cation exchange resin.

18. A method according to claim 17, characterized in that the gel chromatography resin is a cation exchange resin and the $Ca^{2+}$ source is added to the liquid in a concentration effective for increasing the affinity of trypsinogen for binding to the resin, hence the trypsinogen-binding capacity of the resin, preferably by a factor of at least 3, more preferably by a factor of 3 to 6.

19. A method according to claim 17 or 18, characterized in that $Ca^{2+}$ is added at a concentration ranging from 0.5 to 500, preferably 1 - 300, more preferably 5-20 mmol per liter.

20. A method according to any one of claims 17 to 19, characterized in that the method comprises the use of washing and/or eluting buffers and $Ca^{2+}$ is added to each washing and/or eluting buffer at a minimum concentration required for unrestricted activation of trypsinogen to trypsin, preferably at a concentration of at least 0.5, more preferably of at least 1 mM.

**Claims**

1. A method of continuous or semi-continuous refolding of denatured protein, comprising feeding a solution containing denatured protein to a reactor unit, feeding renaturation buffer to said unit, allowing for refolding of the protein, removing denaturing agents comprised in the feed solution containing the denatured protein from said unit, preferably by diafiltration, and draining off from said unit liquid volumes together with refolded protein, wherein feed into and volume removal from the reactor unit is done intermittently or continuously.

2. The method of claim 1, wherein the method is continuous and the solution containing the denatured protein is fed to the reactor unit at a flow rate F1, renaturation buffer is fed to said unit at a flow rate F2, denaturing agents comprised in the feed solution containing the denatured protein are removed from said unit at a flow rate F3, and liquid volumes containing refolded protein are drained off from said unit at a flow rate F4, wherein F1 is adjusted to the refolding rate of the protein.

3. The method of claim 2, wherein the flow rates are adjusted such as to allow for sufficient residence time of the denatured protein for substantially complete, e.g. 95%, refolding of the protein inside the reactor unit.

4. The method according to claim 2 or 3, wherein the flow rates are adjusted such as to maintain a constant or increasing protein concentration within the reactor unit.

5. The method according to any one of claims 1 to 4, wherein the protein is bovine, porcine or human trypsinogen, preferably recombinant human trypsinogen 1.

6. An apparatus for carrying out the method defined in any one of claims 1 to 5, comprising a reactor unit that is equipped with a feed line for feeding a protein-containing solution to the unit, a feed line for feeding a solution for chemical treatment of the protein to the unit, a circulation line connected with a filtration, preferably diafiltration, device for removing undesired ingredients associated with the protein feed, and an outlet for draining off liquid together with treated protein.

7. The apparatus of claim 6, which is operated in a continuous or semi-continuous way.

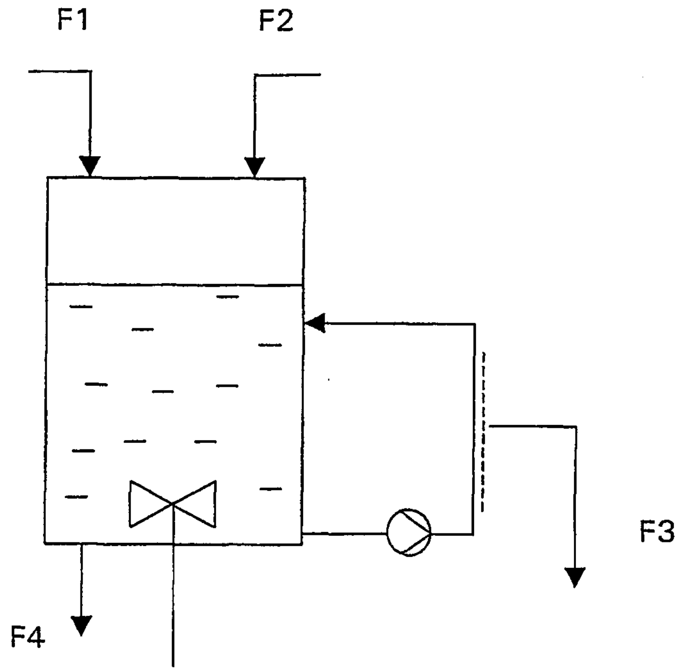


Fig. 1

European Patent Office

# EUROPEAN SEARCH REPORT

Application Number
EP 05 02 1863

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 97/04003 A (AMGEN INC; PURDUE RESEARCH FOUNDATION) 6 February 1997 (1997-02-06) * claims 1-12; figure 1 * ----- | 1-7 | C12N15/57 C12N9/76 C12M1/00 |
| X | KR 00 247 151 B (CHUNG, KWANG HOE KANG) 9 December 1999 (1999-12-09) * abstract * ----- | 1-7 | |
| X | US 4 965 344 A (HERMANN ET AL) 23 October 1990 (1990-10-23) * abstract; claims 1-15 * ----- | 1-7 | |
| X | EP 0 657 466 A (PFIZER INC) 14 June 1995 (1995-06-14) * claim 1 * ----- | 1-7 | |
| E | WO 01/19970 A (ELI LILLY AND COMPANY; HANQUIER, JOSE, MICHAEL; HERSHBERGER, CHARLES,) 22 March 2001 (2001-03-22) * claims 22-26 * ----- | 5 | TECHNICAL FIELDS SEARCHED (IPC) |
| T | LI M ET AL: "In vitro protein refolding by chromatographic procedures" PROTEIN EXPRESSION AND PURIFICATION, ACADEMIC PRESS, SAN DIEGO, CA, US, vol. 33, no. 1, January 2004 (2004-01), pages 1-10, XP004475006 ISSN: 1046-5928 * the whole document * ----- | 1-7 | C12N C12M |

The present search report has been drawn up for all claims

| Place of search | Date of completion of the search | Examiner |
|---|---|---|
| Munich | 30 December 2005 | Stachowiak, O |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 05 02 1863

This annex lists the patent family members relating to the patent documents cited in the above-mentioned European search report.
The members are as contained in the European Patent Office EDP file on
The European Patent Office is in no way liable for these particulars which are merely given for the purpose of information.

30-12-2005

| Patent document cited in search report | Publication date | Patent family member(s) | Publication date |
|---|---|---|---|
| WO 9704003 A | 06-02-1997 | AU 707719 B2 | 15-07-1999 |
| | | AU 6544496 A | 18-02-1997 |
| | | CA 2227318 A1 | 06-02-1997 |
| | | EP 0840745 A2 | 13-05-1998 |
| | | JP 11509551 T | 24-08-1999 |
| | | ZA 9606151 A | 04-02-1997 |
| KR 00247151 B | 15-03-2000 | NONE | |
| US 4965344 A | 23-10-1990 | AU 601190 B2 | 06-09-1990 |
| | | AU 7379687 A | 10-12-1987 |
| | | CA 1338874 C | 21-01-1997 |
| | | DE 3618817 A1 | 10-12-1987 |
| | | EP 0248362 A2 | 09-12-1987 |
| | | JP 2594275 B2 | 26-03-1997 |
| | | JP 62292796 A | 19-12-1987 |
| EP 0657466 A | 14-06-1995 | AT 147753 T | 15-02-1997 |
| | | DE 69401490 D1 | 27-02-1997 |
| | | DE 69401490 T2 | 30-04-1997 |
| | | DK 657466 T3 | 14-07-1997 |
| | | ES 2096416 T3 | 01-03-1997 |
| | | GR 3022653 T3 | 31-05-1997 |
| | | JP 2749269 B2 | 13-05-1998 |
| | | JP 7184650 A | 25-07-1995 |
| WO 0119970 A | 22-03-2001 | AU 7053500 A | 17-04-2001 |

EPO FORM P0459

For more details about this annex : see Official Journal of the European Patent Office, No. 12/82

SEQUENCE LISTING

<110> Mattanovich, Diethard
Katinger, Hermann
Hohenblum, Hubertus
Naschberger, Stefan
Weik, Robert
Polymun Scientific Immunbiologische Forschung GmbH

<120> Method for the manufacture of recombinant trypsin

<130> BEP-4697-PC

<140>
<141>

<150> US 60/177348
<151> 2000-01-24

<160> 8

<170> PatentIn Ver. 2.1

<210> 1
<211> 699
<212> DNA
<213> Human pancreas

<400> 1
gcccctttg atgatgatga caagatcgtt gggggctaca actgtgagga gaattctgtc 60
ccctaccagg tgtccctgaa ttctggctac cacttctgtg gtggctccct catcaacgaa 120
cagtgggtgg tatcagcagg ccactgctac aagtcccgca tccaggtgag actgggagag 180
cacaacatcg aagtcctgga ggggaatgag cagttcatca atgcagccaa gatcatccgc 240
caccccaat acgacaggaa gactctgaac aatgacatca tgttaatcaa gctctcctca 300
cgtgcagtaa tcaacgcccg cgtgtccacc atctctctgc ccaccgcccc tccagccact 360
ggcacgaagt gcctcatctc tggctggggc aacactgcga gctctggcgc cgactaccca 420
gacgagctgc agtgcctgga tgctcctgtg ctgagccagg ctaagtgtga agcctcctac 480
cctggaaaga ttaccagcaa catgttctgt gtgggcttcc ttgagggagg caaggattca 540
tgtcagggtg attctggtgg ccctgtggtc tgcaatggac agctccaagg agttgtctcc 600
tggggtgatg gctgtgccca gaagaacaag cctggagtct acaccaaggt ctacaactac 660
gtgaaatgga ttaagaacac catagctgcc aatagctaa 699

<210> 2
<211> 44
<212> DNA
<213> Artificial Sequence

<220>
<223> Description of Artificial Sequence: PCR primer

<400> 2
catgccatga ctcgcggccc agccggcccc ctttgatgat gatg 44

<210> 3
<211> 48
<212> DNA
<213> Artificial Sequence

<220>
<223> Description of Artificial Sequence: PCR primer

<400> 3

catgccatga ctcgcggccg gctgggcctt agctattggc agctatgg 48

<210> 4
<211> 27
<212> DNA
<213> Artificial Sequence

<220>
<223> Description of Artificial Sequence: PCR primer

<400> 4
ccgctcgagc cccctttgat gatgatg 27

<210> 5
<211> 28
<212> DNA
<213> Artificial Sequence

<220>
<223> Description of Artificial Sequence: PCR primer

<400> 5
gcggatcctt agctattggc agctatgg 28

<210> 6
<211> 32
<212> DNA
<213> Artificial Sequence

<220>
<223> Description of Artificial Sequence: PCR primer

<400> 6
ggaattccat atggcccccct ttgatgatga tg 32

<210> 7
<211> 27
<212> DNA
<213> Artificial Sequence

<220>
<223> Description of Artificial Sequence: PCR primer

<400> 7
gcggatccgc cccctttgat gatgatg 27

<210> 8
<211> 28
<212> DNA
<213> Artificial Sequence

<220>
<223> Description of Artificial Sequence: PCR primer

<400> 8
gcggatcctt agctattggc agctatgg 28